# EUROPEAN PATENT APPLICATION

(11) **EP 1 230 933 A1**
(43) Date of publication of application: **14.08.2002**
(21) Application number: 00963072.4
(22) Date of filing: 02.10.2000
(51) Int. Cl.: A61K 45/00, A61P 17/02

(54) **REMEDIES FOR INTRACTABLE WOUND**

(30) Priority: 12.10.1999 JP 28924799
(71) Applicant: FUJISAWA PHARMACEUTICAL CO., LTD., Osaka-shi Osaka 541-8514 (JP)
(72) Inventor: TAKAKURA, Shoji Fujisawa Pharmaceutical Co., Ltd., Osaka-shi Osaka 541-8514 (JP); MINOURA, Kyoko Fujisawa Pharmaceutical Co., Ltd., Osaka-shi Osaka 541-8514 (JP)
(74) Representative: Gille Hrabal Struck Neidlein Prop Roos
(86) International application number: JP0006873
(87) International publication number: WO0126685

(57) **Abstract**

This invention provides a therapeutic drug for refractory injuries, comprising a substance having a human leucocyte elastase inhibitory activity as an effective ingredient.

## Description

### TECHNICAL FIELD

This invention relates to a therapeutic drug for refractory injuries, comprising a substance having a human leucocyte elastase inhibitory activity as an effective ingredient.

The inventors of this invention have found that a substance having a human leucocyte elastase inhibitory activity is effective for the treatment of refractory injuries and have completed this invention.

### BACKGROUND ART

### INDUSTRIAL APPLICABILITY

This invention is a therapeutic drug for refractory injuries, comprising a substance having a human leucocyte elastase inhibitory activity as an effective ingredient.

### DISCLOSURE OF THE INVENTION

The substance having a human leucocyte elastase inhibitory activity and being usable as an effective ingredient of a therapeutic drug for refractory injuries may be any substance having a human leucocyte elastase inhibitory activity. Furthermore, the substance having a human leucocyte elastase inhibitory activity and being usable in this invention includes not only substances that directly inhibit leucocyte elastase but also substances that indirectly inhibit leucocyte elastase by suppressing the infiltration of leucocytes or by inhibiting the generation of elastase. In other words, various substances having such an activity are known. Not only the known substances but also new substances can also be used if they have the human leucocyte elastase inhibitory. Among these, particularly suitable compounds are exemplified below.

(1) WS7622A mono- or disulfate ester and pharmaceutically acceptable salts thereof: among them, the disodium salt of the WS7622A disulfate ester and the dipotassium salt of the WS7622A disulfate ester are known substances having the following physico-chemical properties respectively (Japanese Laid-open Patent Application No. Hei 4-279600).

Disodium salt of WS7622A disulfate ester
Appearance: colorless crystal
Solubility:
soluble: water, methanol
insoluble: chloroform, n-hexane
Melting point: 257 to 263°C (dec.)
Specific rotation: [α]²³_{D} +37.5° (C=1, methanol)
Molecular formula: C₁₇H₆₁N₉O₁₉S₂Na₂
Elemental analysis:

| | | | | | |
|---|---|---|---|---|---|
| Calcd for C₁₇H₆₁N₉O₁₉S₂Na₂·6H₂O | C 44.30, | H 5.77, | N 9.89, | S 5.03, | Na 3.61 % |
| Found: | C 44.98, | H 5.90, | N 10.06, | S 5.00, | Na 3.98 % |

Molecular weight: FAB-MS m/z 1188 (M+Na)⁺
Thin layer chromatography:

| Stationary phase | Developing solvent | Rf value |
|---|---|---|
| Silica gel | CHCl₃-CH₃OH-H₂O | 0.11 |
| (Merck Art 5715) | (65 : 25 : 4) | |
| | n-butanol-acetic acid-water | 0.29 |

Infrared absorption spectrum:
γ^{KBr}ₘₐₓ: 3360, 2960, 1735, 1660, 1640, 1530, 1500, 1380, 1250, 1200, 1060, 1030, 940, 890 cm⁻¹
¹H Nuclear magnetic resonance spectrum:

| | |
|---|---|
| (400 MHz, D₂O) δ | |
| 7.50 | (1H, s) |
| 7.27 | (1H, s) |
| 7.33-7.24 | (3H, m) |
| 6.94 | (1H, q, J=7Hz) |
| 6.85 | (2H, br d, J=8Hz) |
| 5.53 | (1H, m) |
| 5.37 | (1H, m) |
| 4.80 | (1H, br s) |
| 4.63-4.57 | (2H, m) |
| 4.53 | (1H, m) |
| 4.06 | (1H, m) |
| 3.99 | (1H, d, J=10Hz) |
| 3.56 | (1H, br d, J=14Hz) |
| 3:46 | (1H, m) |
| 2.97 | (3H, s) |
| 2.97-2.88 | (2H, m) |
| 2.72 | (1H, m) |
| 2.59 | (1H, m) |
| 2.51-2.38 | (2H, m) |
| 2.09-1.91 | (4H, m) |
| 1.82-1.60 | (3H, m) |
| 1.77 | (3H, d, J=7Hz) |
| 1.50 | (3H, d, J=6.5Hz) |
| 1.40 | (1H, m) |
| 1.11 | (6H, d, J=7Hz) |
| 0.99 | (3H, d, J=6.5Hz) |
| 0.97 | (3H, d, J=6.5Hz) |

¹³C Nuclear magnetic resonance spectrum:

| | |
|---|---|
| (100 MHz, D₂O) δ | |
| 183.6 | (s) |
| 177.9 | (s) |
| 177.7 | (s) |
| 174.8 | (s) |
| 173.8 | (s) |
| 173.3 | (s) |
| 172.4 | (s) |
| 167.8 | (s) |
| 161.5 | (s) |
| 145.5 | (s) |
| 144.9 | (s) |
| 139.6 | (d) |
| 139.0 | (s) |
| 137.0 | (s) |
| 136.0 | (s) |
| 132.3 | (d) x 2 |
| 131.0 | (d) x 2 |
| 129.6 | (d) |
| 127.4 | (d) |
| 125.9 | (d) |
| 77.4 | (d) |
| 75.1 | (d) |
| 63.8 | (d) |
| 62.7 | (d) |
| 59.1 | (d) |
| 55.9 | (d) |
| 54.9 | (d) |
| 51.9 | (d) |
| 41.9 | (t) |
| 37.2 | (d) |
| 36.9 | (t) |
| 34.1 | (q) |
| 32.3 | (d) |
| 31.9 | (t) |
| 31.8 | (t) |
| 31.2 | (t) |
| 27.5 | (t) |
| 23.7 | (t) |
| 21.7 | (q) |
| 21.4 | (q) x 2 |
| 21.3 | (q) |
| 21.1 | (q) |
| 15.5 | (q) |

### Amino acid analysis

The disodium salt (1 mg) of the WS7622A disulfate ester was hydrolyzed in 6N hydrochloric acid (1 ml) at 110°C for 20 hours, and dried under reduced pressure to obtain a mixture. The mixture was measured by Hitachi 835 Automatic Amino Acid Analyzer. Type H (Wako code: 013-08391) and type B (Wako code: 016-08641) of Wako Pure Chemical Industries, Ltd. were used as standard amino acid samples.

As a result, threonin, valine, phenyl alanine, ornithine, ammonia and several kinds of unknown ninhydrin positive components were detected.

The following formula is proposed as a partial chemical structural formula of the disodium salt of the WS7622A disulfate ester. 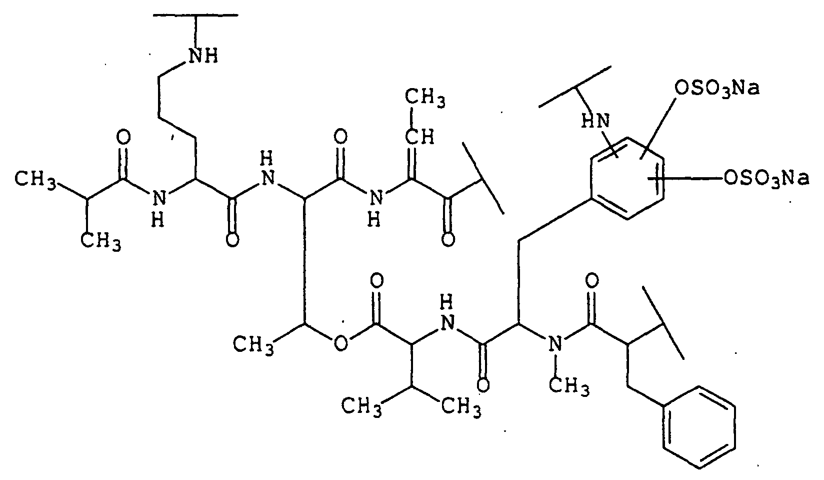 Dipotassium salt of the WS7622A disulfate ester
Appearance: colorless amorphous powder
Solubility:
soluble: water, methanol
insoluble: chloroform, n-hexane
Melting point: 230 to 237°C (dec.)
Specific rotation: [α]²³_{D} +34° (C=1, methanol)
Molecular formula: C₁₇H₆₁N₉O₁₉S₂K₂
Elemental analysis:

| | | | | | |
|---|---|---|---|---|---|
| Calcd for C₁₇R₆₁N₉O₁₉S₂K₂ · 6H₂O | C 43.21, | H 5.63, | N 9.65, | S 4.91, | K 5.99 % |
| Found: | C 43.96, | H 5.44, | N 9.97, | S 5.09, | K 4.49 % |

Molecular weight: FAB-MS m/z 1236 (M+K)⁺
Thin layer chromatography:

| Stationary phase | Developing solvent | Rf value |
|---|---|---|
| Silica gel | CHCl₃-CH₃OH-H₂O | 0.13 |
| (Merck Art 5715) | (65 : 25 : 4) | |

Infrared absorption spectrum:
γ^{KBr}ₘₐₓ: 3360, 2960, 1735, 1660, 1640, 1530, 1500, 1405, 1380, 1250, 1200, 1050, 1030, 910, 890 cm⁻¹
¹H Nuclear magnetic resonance spectrum:

| | |
|---|---|
| (400 MHz, D₂O) δ | |
| 7.52 | (1H, s) |
| 7.28 | (1H, s) |
| 7.34-7.25 | (3H, m) |
| 6.96 | (1H, q, J=7Hz) |
| 6.87 | (2H, br d, J=8Hz) |
| 5.56 | (1H, m) |
| 5.40 | (1H, m) |
| 4.84 | (1H, br s) |
| 4.70-4.55 | (3H, m) |
| 4.10 | (1H, m) |
| 4.03 | (1H, m) |
| 3.60 | (1H, br d, J=14Hz) |
| 3.50 | (1H, m) |
| 3.00 | (3H, s) |
| 3.00-2.85 | (2H, m) |
| 2.76 | (1H, m) |
| 2.62 | (1H, m) |
| 2.55-2.40 | (2H, m) |
| 2.12-1.95 | (4H, m) |
| 1.90-1.65 | (3H, m) |
| 1.79 | (3H, d, J=7Hz) |
| 1.53 | (3H, d, J=6.5Hz) |
| 1.45 | (1H, m) |
| 1.14 | (6H, d, J=7Hz) |
| 1.02 | (3H, d, J=6.5Hz) |
| 1.00 | (3H, d, J=6.5Hz) |

### Amino acid analysis

The dipotassium salt (1 mg) of the WS7622A disulfate ester was hydrolyzed in 6N hydrochloric acid (1 ml) at 110°C for 20 hours, and dried under reduced pressure to obtain a mixture. The mixture was measured by Hitachi 835 Automatic Amino Acid Analyzer. Type H (Wako code: 013-08391) and type B (Wako code: 016-08641) of Wako Pure Chemical Industries, Ltd. were used as standard amino acid samples.

As a result, threonin, valine, phenyl alanine, ornithine, ammonia and several kinds of unknown ninhydrin positive components were detected.

The following formula is proposed as a partial chemical structural formula of the dipotassium salt of the WS7622A disulfate ester.

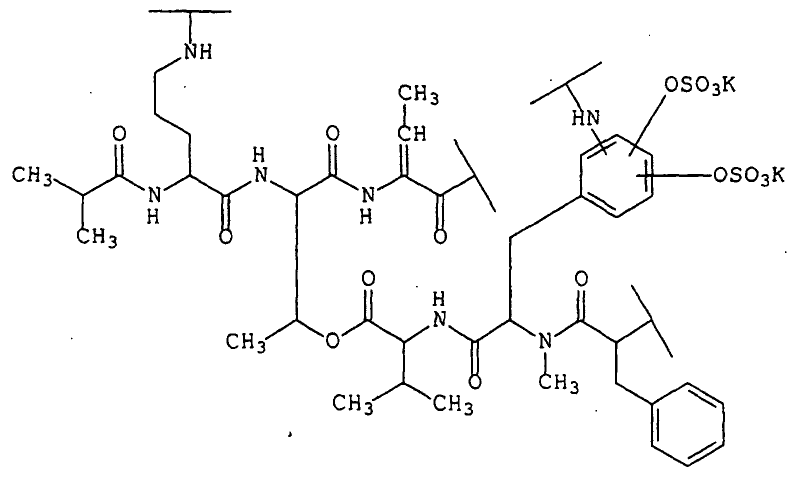

Pharmaceutically acceptable salts of the WS7622A mono- or disulfate ester may include a mono- or disalt with an inorganic or organic base, for example, an alkali metal salt (e.g., sodium salt, potassium salt, etc.), an alkaline earth metal salt (e.g., calcium salt, etc.), an ammonium salt, an ethanolamine salt, a triethylamine salt, a dicyclohexylamine salt, a pyridine salt, etc.

The WS7622A substance, a starting substance for the synthesis of the above-mentioned WS7622A mono- or disulfate ester, also has the human leucocyte elastase inhibitory activity and can be used as a therapeutic drug for refractory injuries. The substance is known as a substance having the following physico-chemical properties (Japanese Laid-open Patent Application No. Hei 3-218387 and Japanese Laid-open Patent Application No. Hei 4-279600).

### Physico-chemical properties of the WS7622A substance

Appearance: colorless prism crystal
Property of substance: acidic
Color reaction:
Positive: cerium sulfate reaction, iodine vapor reaction, ferric chloride reaction
Negative: ninhydrin reaction, Molisch reaction, Dragendorff reaction
Solubility:
soluble: methanol, ethanol, n-butanol
slightly soluble: chloroform, acetone, ethyl acetate
insoluble: water, n-hexane
Thin layer chromatography (TLC):
Chloroform-methanol (5 : 1, v/v)
Rf value 0.51
Acetone-methanol (10 : 1)
Rf value 0.62
   (Kiesel gel 60F₂₅₁ silica gel plate, Merck)
Melting point: 250 to 252°C (dec.)
Specific rotation: [α]²³_{D} +36° (C=1, methanol)
UV spectrum:
λ^{MeOH}ₘₐₓ 287 nm (ξ = 3600)
λ^{MeOH-HCl}ₘₐₓ 287 nm
λ^{MeOH-NaOH}ₘₐₓ 298 nm
Molecular formula: C₁₇H₆₃N₉O₁₃
Elemental analysis:

| | | | |
|---|---|---|---|
| Calcd for C₁₇H₆₃N₉O₁₃·2H₂O | C 56.56, | H 6.77, | N 12.63 % |
| Found: | C 56.65, | H 6.62, | N 12.27 % |

Molecular weight: FAB-MS m/z 984 (M+Na)⁺
Infrared absorption spectrum:
γ^{KBr}ₘₐₓ: 3400, 3300, 3060, 2980, 2940, 1735, 1710, 1690, 1670, 1660, 1640, 1540, 1520, 1470, 1380, 1330, 1300, 1260, 1220, 1200, 1160, 1130, 1090, 1000, 980, 940, 920 cm⁻¹
¹H Nuclear magnetic resonance spectrum:

| | |
|---|---|
| (400 MHz, CD₃OD) δ | |
| 7.22-7.09 | (3H, m) |
| 6.88-6.77 | (3H, m) |
| 6.74 | (1H, s) |
| 6.46 | (1H, s) |
| 5.46 | (1H, m) |
| 5.18 | (1H, s) |
| 4.85 | (1H, s) |
| 4.77 | (1H, m) |
| 4.65 | (1H, m) |
| 4.50 | (1H, m) |
| 3.96 | (1H, m) |
| 3.91 | (1H, d, J=9Hz) |
| 3.60-3.47 | (2H, m) |
| 3.03 | (1H, m) |
| 2.90 | (3H, s) |
| 2.86 | (1H, m) |
| 2.59-2.49 | (2H, m) |
| 2.39 | (1H, m) |
| 2.29-2.16 | (2H, m) |
| 2.00 | (1H, m) |
| 1.84 | (1H, m) |
| 1.74 | (3H, d, J=6Hz) |
| 1.72-1.53 | (4H, m) |
| 1.44 | (3H, d, J=6Hz) |
| 1.12 | (1H, m) |
| 1.10 | (6H, d, J=6Hz) |
| 0.99 | (3H, d, J=6Hz) |
| 0.94 | (3H, d, J=6Hz) |

¹³C Nuclear magnetic resonance spectrum:

| | |
|---|---|
| (100 MHz, CD₃OD) δ | |
| 179.7 | (s) |
| 176.3 | (s) |
| 174.7 | (s) |
| 173.3 | (s) |
| 172.4 | (s) |
| 171.4 | (s) |
| 170.3 | (s) |
| 165.8 | (s) |
| 160.2 | (s) |
| 145.7 | (s) |
| 145.6 | (s) |
| 137.5 | (s) |
| 134.0 | (d) |
| 131.4 | (s) |
| 130.6 | (d) x 2 |
| 129.8 | (s) |
| 129.1 | (d) x 2 |
| 129.1 | (s) |
| 127.6 | (d) |
| 119.1 | (d) |
| 118.0 | (d) |
| 76.0 | (d) |
| 73.4 | (d) |
| 63.1 | (d) |
| 61.4 | (d) |
| 57.1 | (d) |
| 53.6 | (d) |
| 52.7 | (d) |
| 50.5 | (d) |
| 39.9 | (t) |
| 36.1 | (t) |
| 35.8 | (d) |
| 31.8 | (q) |
| 31.0 | (t) |
| 30.8 | (d) |
| 29.9 | (t) |
| 29.7 | (t) |
| 25.2 | (t) |
| 22.3 | (t) |
| 20.2 | (q) |
| 20.0 | (q) x 2 |
| 19.7 | (q) |
| 19.5 | (q) |
| 13.3 | (q) |

### Amino acid analysis

WS7622A(1 mg) was hydrolyzed in 6N hydrochloric acid (1 ml) at 110°C for 20 hours, and dried under reduced pressure to obtain a mixture. The mixture was measured by Hitachi 835 Automatic Amino Acid Analyzer. Type H (Wako code: 013-08391) and type B (Wako code: 016-08641) of Wako Pure Chemical Industries, Ltd. were used as standard amino acid samples.

As a result, threonin, valine, phenyl alanine, ornithine, ammonia and several kinds of unknown ninhydrin positive components were detected.

The following formula is proposed as a partial chemical structural formula of the WS7622A.

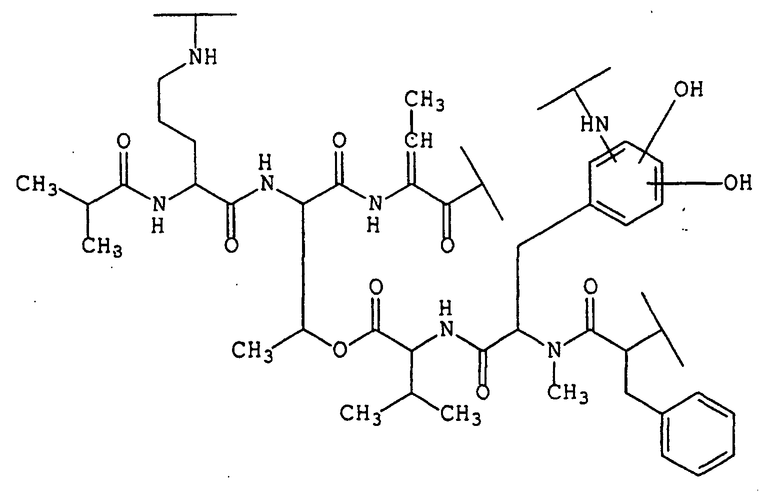

Salts of the WS7622A substance may include a salt with an inorganic or organic base, for example, an alkali metal salt (e.g., sodium salt, potassium salt, etc.), an alkaline earth metal salt (e.g., calcium salt, etc.), an ammonium salt, an ethanolamine salt, a triethylamine salt, a dicyclohexylamine salt, etc.

Similarly, WS7622B, WS7622C and WS7622D substances and their derivatives (Japanese Laid-open Patent Application No. Hei 3-218387), having the human leucocyte elastase inhibitory activity, can also be used as therapeutic drugs for refractory injuries.

The above-mentioned WS7622A substance (similarly, WS7622B, WS7622C and WS7622D substances) can be produced by culturing the streptomyces resistomycificus No. 7622 strain, for example. The fungal strain was deposited with National Institute of Bioscience and Human-Technology, an international depository authority on the Budapest Treaty, under the deposit number FERM BP-2306.

(2) Trifluoromethylketone derivative represented by the following formula: 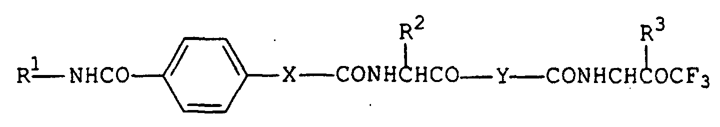 in which R¹ is lower alkyl having one or two substituents selected from a group consisting of carboxy, esterified carboxy and di-lower alkylcarbamoyl; phenyl (lower) alkyl which may have halogen, amino or nitro at the phenyl moiety and may have carboxy or esterified carboxy at the alkyl moiety; halophenyl; morpholino; or morpholino(lower)alkyl,

R² and R³ are each lower alkyl,
X is - or -NH-,
Y is 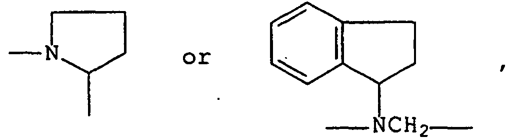 and a pharmaceutically acceptable salt thereof.

(3) Trifluoromethylketone derivative represented by the following formula: 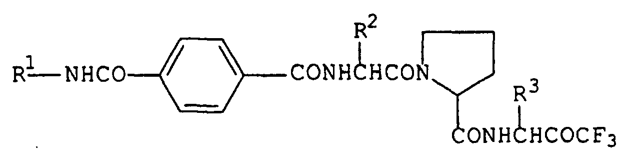 in which R¹ to R³ are the same as those of the above-mentioned compound (2), and a pharmaceutically acceptable salt thereof.

(4) 3(RS)-[[4-(carboxymethylaminocarbonyl)phenylcarbonyl]-L-valyl-L-prolyl]amino-1,1,1-trifluoro-4-methyl-2-oxopentane or a sodium salt thereof

The compounds described at the above items (2) to (4) are known compounds described in Japanese Laid-open Patent Application No. Hei 4-297446. In addition, pharmaceutically acceptable salts of the compounds described at the items (2) to (4) may include a salt with an inorganic or organic base, for example, an alkali metal salt (e.g., sodium salt, potassium salt, etc.), an alkaline earth metal salt (e.g., calcium salt, etc.), an ammonium salt, an ethanolamine salt, a triethylamine salt, a dicyclohexylamine salt, etc., and an organic or inorganic acid addition salt, for example, methanesulfonate, hydrochloride, sulfate, nitrate, phosphate, etc.

Suitable examples of the above-mentioned definitions are explained in detail as follows.

The term "lower" is intended to means 1 to 6 carbon atoms, unless otherwise indicated.

Suitable examples of "halogen" may include fluorine, chlorine, bromine and iodine.

Suitable "lower alkyl" may include a straight or branched alkane residue having 1 to 6 carbon atoms, such as methyl, ethyl, propyl, isopropyl, butyl, isobutyl, t-butyl, pentyl, neo-pentyl, hexyl and the like, preferably those having 1 to 4 carbon atoms.

Suitable "esterified carboxy" may be alkyl ester, that is, alkoxycarbonyl, for example, lower alkoxycarbonyl (e.g. methoxycarbonyl, ethoxycarbonyl, propoxycarbonyl, butoxycarbonyl, tert-butoxycarbonyl, etc.) and phenyl(lower)alkyl ester, that is, phenyl(lower)alkoxy carbonyl, for example, benziloxycarbonyl, and benzoyl(lower)alkyl ester, that is, benzoyl(lower)alkoxy carbonyl, for example, benzoylmethoxycarbonyl, etc.

Suitable "lower alkylene" may include methylene, ethylene, propylene, isopropylene, etc.

Suitable "di-lower alkylcarbamoyl" may include N,N-dimethylcarbamoyl, N,N-diethylcarbamoyl, etc.

(5) FR901451 substance having the following physico-chemical properties and a pharmaceutically acceptable salt thereof
Appearance: white powder
Color reaction:
Positive: cerium sulfate, iodine vapor, Ehrlich, ninhydrin
Negative: Molisch
Solubility: soluble: water, methanol, dimethyl sulfoxide
hardly soluble: acetone
insoluble: ethyl acetate
Melting point: 243 to 245°C (dec.)
Specific rotation: [α]²³_{D} -15° (C=0.65, H₂O)
UV absorption spectrum: λ^{MeOH}ₘₐₓ nm (ξ) 275 = (4300) 281 (4500), 290 (3900)
Molecular formula: C₆₀H₇₉N₁₃O₁₈
Elemental analysis:

| | | | |
|---|---|---|---|
| Calcd for C₆₀H₇₉N₁₃O₁₈ · 10H₂O | C 49.68, | H 6.88, | N 12.55 % |
| Found: | C 49.95, | H 6.28, | N 12.42 % |

Molecular weight: FAB-MS m/z 1270 (M+H)⁺
Thin layer chromatography:

| Stationary phase | Developing solvent | Rf value |
|---|---|---|
| Silica gel | CHCl₃ : MeOH: NH₄OH | 0.60 |
| (Merck) | (15 : 11 : 5) | |
| RP-18 | 70% hydrous methanol | 0.32 |
| (Merck) | | |

FT Infrared absorption spectrum:
γ^{KBr}ₘₐₓ: 3390, 3070, 2970, 2880, 1740, 1660, 1530, 1450, 1410, 1380, 1350, 1250, 1190, 1110, 1080, 1010, 750, 700, 670, 660, 620, 600 cm⁻¹
¹H Nuclear magnetic resonance spectrum:

| | |
|---|---|
| (400 MHz, D₂O) δ | |
| 7.70 | (1H, d, J=7Hz) |
| 7.52 | (1H, d, J=7.5Hz) |
| 7.44-7.23 | (7H, m) |
| 7.22 | (1H, s) |
| 5.59 | (1H, q, J=7Hz) |
| 4.94 | (1H, t, J=4.5Hz) |
| 4.85-4.74 | (3H, m) |
| 4.58 | (1H, dd, J=6Hz, 10Hz) |
| 4.45-4.35 | (3H, m) |
| 4.30 | (1H, dd, J=4Hz, 7Hz) |
| 4.07 | (1H, m) |
| 3.99 | (1H, dd, J=10Hz, 4.5Hz) |
| 3.66-3.50 | (3H, m) |
| 3.44-3.25 | (4H, m) |
| 3.16-2.93 | (4H, m) |
| 2.87 | (1H, d, J=18Hz) |
| 2.80-2.68 | (2H, m) |
| 2.56-2.48 | (2H, m) |
| 2.08 | (1H, dd, J=16Hz, 4Hz) |
| 1.87-1.53 | (9H, m) |
| 1.43 | (3H, d, J=7Hz) |
| 1.30 | (3H, d, J=6.5Hz) |
| 1.45-1.17 | (4H, m) |
| 0.95 | (3H, d, J=6Hz) |
| 0.84 | (3H, d, J=6Hz) |

¹³C Nuclear magnetic resonance spectrum:

| | | | |
|---|---|---|---|
| (100 MHz, D₂O) δ | | | |
| 177.2 (s) | 130.0 (d) x 2 | 56.0 (d) | 31.4 (t) |
| 176.5 (s) | 129.8 (d) x 2 | 54.1 (d) | 28.8 (t) |
| 174.6 (s) | 128.5 (d) | 53.8 (d) | 26.6 (t) |
| 174.2 (s) | 127.8 (d) | 53.2 (d) | 25.1 (d) |
| 174.0 (s) | 125.5 (d) | 53.1 (d) | 23.2 (q) |
| 173.2 (s) | 123.2 (d) | 52.9 (d) | 23.2 (t) |
| 173.0 (s) | 120.9 (d) | 52.8 (d) | 23.1 (t) |
| 172.8 (s) | 118.7 (d) | 49.5 (d) | 20.8 (q) |
| 172.6 (s) | 113.1 (d) | 48.6 (t) | 19.4 (q) |
| 172.5 (s) | 108.8 (s) | 40.1 (t) | 18.3 (q) |
| 172.1 (s) | 73.3 (d) | 39.6 (t) | |
| 171.7 (s) | 69.7 (d) | 39.4 (t) | |
| 171.4 (s) | 64.3 (d) | 38.9 (t) | |
| 170.3 (s) | 62.1 (d) | 35.3 (t) | |
| 137.2 (s) | 60.9 (d) | 34.8 (t) | |
| 136.0 (s) | 57.1 (d) | 31.7 (t) | |

The above-mentioned FR90145 substance is known as a substance produced from the FR90145 substance producing fungus of the flexibacter genus (for example, International Publication No. WO93/02203). In addition, the flexibacter sp No. 758 strain of the producing fungus was deposited with National Institute of Bioscience and Human-Technology, an international depository authority on the Budapest Treaty, under the deposit number FERM BP-3420.

Furthermore, pharmaceutically acceptable salts of the above-mentioned FR90145 substance may be the same as the pharmaceutically acceptable salts of the compounds described at the above-mentioned items (2) to (4).

In addition to those described above, examples of substances having the elastase inhibitory activity may include α1-antitrypsin, SLP1 (Secretory Leukocyte Protease Inhibitor) (American Review of Respiratory Disease Vol. 147, 1993, P442-446), urinastatin, colchicine, erythromycin, clarithromycin, IC1200, 800, ONO-5046 (American Journal of Respiratory and Critical Care Medicine Vol. 153, P391-397), antielastase antibody, etc.

Examples of refractory injuries in accordance with this invention may include ulcers at skin (e.g. decubitus (bedsore), foot ulcers associated with diabetes, etc.), ulcers at feet, stomach, cornea, etc. and the like. The therapeutic drug for refractory injuries in accordance with this invention is particularly suited for the treatment of refractory skin ulcers, such as foot ulcers associated with diabetes, among the above-mentioned ulcers.

The therapeutic drug for refractory injuries in accordance with this invention is usually used as external preparations (e.g. lotions, ointments, plasters, liniments, aerosols, suspensions, emulsions, etc.) in the case of refractory skin ulcers, for example. In addition, the therapeutic drug can be used in the forms of conventional pharmaceutical preparations, such as powders, fine granules, granules, tablets, dragees, injection solutions, insufflations, microcapsules, capsules, suppositories, solutions, syrups, etc.

If necessary, there may be included in the above preparations diluents, disintegrating agents (e.g. sucrose, starch, crystalline cellulose, L-hydroxypropylcellulose, synthetic aluminum silicate, etc.), binders (e.g. cellulose, methylcellulose, hydroxypropylcellulose, hydroxypropylmethylcellulose, polypropylpyrrolidone, polyvinylpyrrolidone, gelatin, gum Arabic, polyethylene glycol, etc.), coloring agents, sweeteners, lubricants (e.g. magnesium stearate, etc.) and the like.

While the dosage of the therapeutic drug for refractory injuries in accordance with this invention varies depending on the condition and the like of each patient to be treated, in the case of external administration, a dose of about 0.001-10% of the substance having a human leucocyte elastase inhibitory activity or a pharmaceutically acceptable salt thereof should be used generally.

Next, the effects of this invention are described by using a test example.

### Test example (diabetic rat foot ulcer curing action)

### Purpose:

The action of the compound (applied) in accordance with this invention on a foot ulcer induced by acetic acid was examined by using normal and diabetic rats.

### Compound used for the test:

Sodium salt of 3(RS)-[[4-(carboxymethylaminocarbonyl) phenylcarbonyl]-L-valyl-L-prolyl]amino-1,1,1-trifluoro-4-methyl-2-oxopentane (FR136706)

### Method:

Diabetes was induced in each of a seven-week-old male SD rats by intravenously administrating 60 mg/kg streptozotocin (STZ) to its tail. Fourteen days after the administration of STZ, 20 µl glacial acetic acid was administered into the skin of the left foot instep of each of the diabetic rats and control rats of the same age while anesthetized using ether, thereby causing necrosis at the portion. In the case when the necrotic cuticle of the skin remained two days after the necrosis, the cuticle was removed surgically. Then, the administration of FR136706 (0.2% solution in PEG (polyethylene glycol) 400) was started (50 µl to the affected portion). PEG400 was administered to the control group in a similar way.

In a period between two days and 25 days after the administration of acetic acid, swelling scores (0: no swelling, 1: slight swelling, 2: intermediate swelling, 3: significant swelling) was checked visually, and the major axis length and the minor axis length of each ulcer was measured with vernier calipers. The area of each ulcer was calculated from the major axis length and the minor axis length thereof.

### Result:

The swelling scores of the normal rats were highest on the measurement start day. Then, the rats were recovered and their scores became zero 22 days after the administration of the acetic acid. On the other hand, in the case of the diabetic rats, the peaks of the swelling scores were found seven days after the administration of the acetic acid. Although the rats were recovered gradually after that, the progress of the recovery was slower than that of the normal rats. FR136706 did not act on the normal rats, but promoted the recovery of the diabetic rats.

The swelling areas of the diabetic rats were larger than those of the normal rats, and the contraction of the areas of the diabetic rats was slower than that of the normal rats. FR136706 did not act on the normal rats, but it was recognized that FR136706 tended to promote the contraction of the ulcer areas of the diabetic rats.

| Action on foot ulcer models | | | | | | | | | |
|---|---|---|---|---|---|---|---|---|---|
| Animal | Specimen | Dosage (%) | Score | | | | | | |
| | | | Swelling score after administration of acetic acid | | | | | | |
| | | | After 2 days | After 8 days | After 11 days | After 15 days | After 18 days | After 22 days | After 25 days |
| Normal rat | PEG 400 | | 2.5 ±0.2 (6) | 2.3 ±0.2 (6) | 1.8 ±0.2 (6) | 1.0 ±0.0 (6) | 0.3 ±0.2 (6) | 0.0 ±0.0 (6) | 0.0 ±0.0 (6) |
| | FRI 136706 | 0.2 | 2.5 ±0.2 (6) | 2.0 ±0.2 (6) | 1.5 ±0.2 (6) | 1.0 ±0.0 (6) | 0.5 ±0.2 (6) | 0.0 ±0.0 (6) | 0.0 ±0.0 (6) |
| Diabetic rat | PEG 400 | | | | * | ** | ** | ** | ** |
| | | | 2.2 ±0.2 (6) | 2.8 ±0.2 (6) | 2.7 ±0.2 (6) | 2.2 ±0.3 (6) | 2.0 ±0.3 (6) | 1.7 ±0.3 (6) | 1.5 ±0.2 (6) |
| | FRI 136706 | 0.2 | 2.2 ±0.2 (6) | 2.8 ±0.2 (6) | 2.5 ±0.2 (6) | 1.5 ±0.2 (6) | 1.5 ±0.2 (6) | 1.2 ±0.2 (6) | & 0.7 ±0.2 (6) |
| Average ± standard error (n) | | | | | | | | | |

| | | | | | | | | | |
|---|---|---|---|---|---|---|---|---|---|
| &, &&: significant at 5% and 1% respectively (Wilcoxon Rank Sum Test) [Score] [Scores of PEG400 group of diabetic rats and FRI136706 0.2% group of diabetic rats on each measurement day] *, **: significant at 5% and 1% respectively (Wilcoxon Rank Sum Test) [Score] [Scores of PEG400 group of normal rats and PEG400 group of diabetic rats on each measurement day] | | | | | | | | | |

| Action on foot ulcer models | | | | | | | | | |
|---|---|---|---|---|---|---|---|---|---|
| Animal | Specimen | Dosage (%) | Ulcer area (mm²) after administration of acetic acid | | | | | | |
| | | | After 2 days | After 8 days | After 11 days | After 15 days | After 18 days | After 22 days | After 25 days |
| Normal rat | PEG 400 | | 58.88 | 70.29 | 52.61 | 24.99 | 1.51 | 0.00 | 0.00 |
| | | | ±4.31 | ±6.13 | ±6.36 | ±2.82 | ±0.78 | ±0.00 | ±0.00 |
| | | | (6) | (6) | (6) | (6) | (6) | (6) | (6) |
| | FRI 136706 | 0.2 | 58.37 | 71.42 | 53.21 | 18.32 | 0.69 | 0.00 | 0.00 |
| | | | ±6.08 | ±8.43 | ±5.11 | ±4.55 | ±0.36 | ±0.00 | ±0.00 |
| | | | (6) | (6) | (6) | (6) | (6) | (6) | (6) |
| Diabetic rat | PEG 400 | | | * | ** | ** | ** | | |
| | | | 69.28 | 95.58 | 86.03 | 51.63 | 23.38 | 15.94 | 11.05 |
| | | | ±5.33 | ±8.62 | ±7.71 | ±6.12 | ±1.42 | ±3.90 | ±1.68 |
| | | | (6) | (6) | (6) | (6) | (6) | (6) | (6) |
| | FRI 136706 | 0.2 | 69.17 | 91.77 | 72.38 | 41.00 | 16.10 | 12.08 | 6.99 |
| | | | ±5.64 | ±6.16 | ±10.37 | ±10.80 | ±6.43 | ±3.73 | ±1.71 |
| | | | (6) | (6) | (6) | (6) | (6) | (6) | (6) |
| Average ± standard error (n) | | | | | | | | | |

| | | | | | | | | | |
|---|---|---|---|---|---|---|---|---|---|
| *, **: significant at 5% and 1% respectively (Student-t or Aspin-Welch) [Ulcer area] [PEG400 group of normal rats and PEG400 group of diabetic rats on each measurement day] | | | | | | | | | |

## Claims

1. A therapeutic drug for refractory injuries, comprising a substance having a human leucocyte elastase inhibitory activity as an effective ingredient.
